# EUROPEAN PATENT SPECIFICATION

(11) **EP 2 345 392 B1**
(45) Date of publication and mention of the grant of the patent: **09.05.2018**
(21) Application number: 11151191.1
(22) Date of filing: 17.01.2011
(51) Int. Cl.: A61F 2/46, A61F 2/00

(54) **Straight acetabular cup impactor**
Gerades Einsetzwerkzeug für Hüftpfanne
Impacteur droit de coupule acétabulaire

(43) Date of publication of application: 20.07.2011
(73) Proprietor: Greatbatch Medical SA, 2534 Orvin (CH)
(72) Inventor: Burgi, Jonas, 2740 Moutier (CH); Claude, Sarah, 25500 La Chenalotte (FR)
(74) Representative: Garratt, Peter Douglas

(56) References cited:
- EP-A1- 0 453 694
- EP-A1- 1 438 936
- WO-A1-95/11641
- WO-A1-2009/136284
- US-A1- 2002 177 854

## Description

### REFERENCE TO CO-PENDING APPLICATION

Priority is claimed to U.S. provisional patent application serial number 61/295,215, filed on January 15, 2010.

### FIELD OF THE INVENTION

The invention relates to surgical tools for aiding a surgeon installing orthopedic prostheses in patients.

### BACKGROUND OF THE INVENTION

A total hip replacement is a reconstructive surgical procedure performed by an orthopedic surgeon and the surgeon's team. A total hip replacement involves the placement of an acetabular cup in a patient's acetabular socket, and the replacement of the patient's femoral neck with a prosthesis which terminates in a ball specifically designed to be positioned in the acetabular cup. Other surgical procedures may require the application of an acetabular cup or other device applied to a patient.

For example during such acetabular cup procedures, the patient's acetabular socket is reamed out by the surgeon so as to create an enlarged recess to receive the acetabular cup. After the acetabular socket has been reamed, the cup is inserted into the recess and adjusted as necessary to the proper angular orientation. Once deployed, the cup provides a new socket and lining for the patient's acetabulum.

Insertion and placement of the cup by the surgeon is effected either by hand or by use of a hand tool that grips the cup. Once the cup is properly positioned in the acetabulum, the cup can be fixed in the desired location by various means - bone screws, medically acceptable adhesives, or combinations thereof. In many instances, the fixation means include passing bone screws through the cup and into pre-drilled screw holes in the pelvic bone. The bone screws, which are optional, serve to hold the acetabular cup in the acetabulum until bone ingrowth provides permanent fixation.

In one acceptable medical method, the cup is properly positioned in the acetabulum by implantation. One conventional implantation method is, after obtaining proper alignment, to impact an acetabular cup into place. While impacting the acetabular cup, the surgeon listens for a change in pitch as the cup seats down. The surgeon then probes screw holes to determine if a gap between the cup and the bone is present. If a gap is present, the surgeon further impacts the cup into the acetabulum.

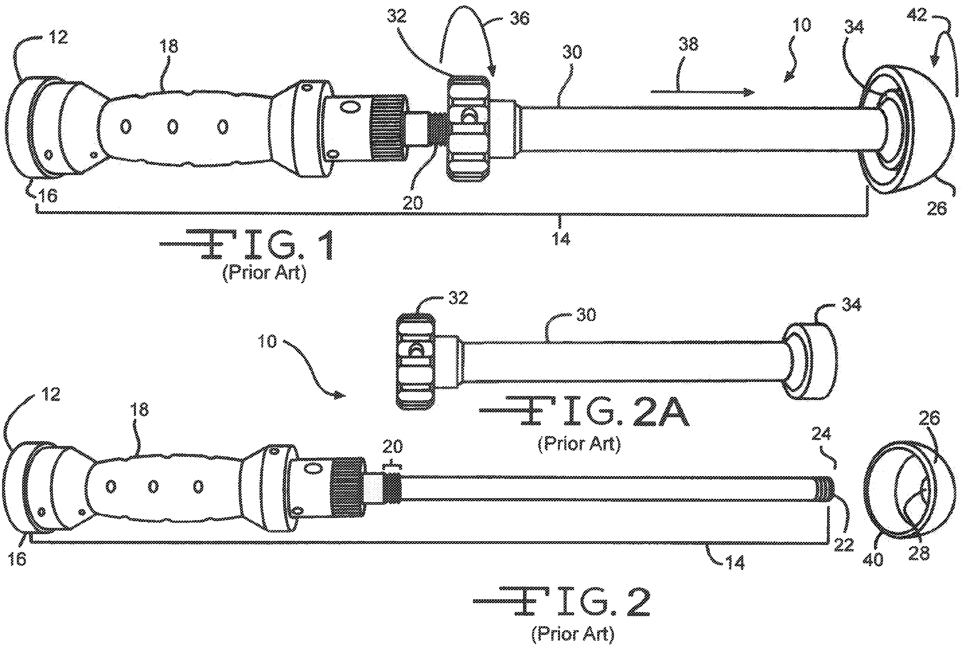

As illustrated in Figs. 1 and 2, a conventional spindle-type surgical tool holder has a strike plate 12 integrally connected to a body's 14 proximal end 16. Extending from the strike plate 12 and positioned over a proximal area of the body portion 14 is a fixed handle 18. The fixed handle 18 has a length that allows a surgeon to hold the tool holder 10, in one embodiment with one hand, and in an alternative embodiment with two hands. Whatever the fixed handle's 18 length, extending there from on the body 14 is an impactor thread section 20. At the body portion's distal end 22 is a tool thread section 24.

The tool thread section 24 threadingly interconnects to a surgical implant device (a.k.a., medical attachment) 26, for example, and not limited to, an acetabular cup, through a threaded aperture 28 (Fig. 2). That means the implant device 26 is directly connected to the body 14 and the strike plate 12. To ensure the surgical implant device 26 is properly secured to the tool thread section 24, the prior art device 10 uses a rotate handle device 30 (Fig. 2A).

The rotate handle 30 is positioned in the spacing between the tool thread section 24 and the impactor thread section 20. At its proximal end, the rotate handle device 30 has a rotating threaded section 32 and at its distal end, an implant support 34. The rotating threaded section 32 has threads that mate with the impactor thread section 20. When the rotating threaded section 32 is rotated clockwise (illustrated by arrow 36 at Fig. 1), (a) the rotating threaded section 32 pushes (illustrated by arrow 38) the rotate handle 30 and the implant support surface 34 toward the surgical implant device's interior surface 40 (Fig. 2); and (b) simultaneously, the rotating threaded section 32, through a conventional lock-nut structure, rotates the surgical implant device 26 counterclockwise (arrow 42 at Fig. 1). This movement results in the surgical implant 26 being pushed toward the implant support 34. Collectively, the clock-wise rotating threaded section 32 is designed to securely position the surgical implant 26 against the implant support 34 to inhibit dislodging of the surgical implant device 26 from the spindle-type tool holder 10 when the surgeon impacts the tool holder.

However, when the surgeon impacts the strike plate 12 there is a chance that the surgical implant 26 can disconnect from the tool holder 10. This could occur when the tool holder's 10 threaded section 24 or the implant's 26 corresponding threaded section 28 are damaged, possibly by the act of impaction. Accordingly, what is needed is a firm fixation of the surgical implant 26 during impaction that provides minimal damage to the implant's 26 threads 28. That desired product is achieved with the current invention.

EP-A-1438936 describes a device having a manipulation handle provided with a gripping head in its distal part and a surface for applying an impact force in its proximal part.

WO 2009/136284 describes a prosthetic acetabular cup inserter and impactor.

US 2002/177854 describes a prosthetic implant and a surgical tool for gripping the prosthetic implant during a surgical procedure.

EP-A-0453694 describes a cup designed to be fixed without cement in total hip prosthesis.

WO 95/11641 describes an acetabular cup positioning tool and a method of positioning the acetabular cup using the tool.

### SUMMARY OF THE INVENTION

The present invention provides a surgical tool handling device as defined in claim 1. The present invention also provides a kit comprising: a medical instrument having a threaded aperture; and a surgical tool handling device according to the invention.

The surgical tool handling device of the present invention has both non-moving and moving components during connection to an implant after the implant cup seats. However, during impaction, the entire surgical device is static or rigid. Having both moving and non-moving parts ensures a stable threaded connection between the surgical tool and the implantable cup.

### BRIEF DESCRIPTION OF THE DRAWINGS

Fig. 1 is a prior art illustration of an alternative spindle-type surgical tool.
Fig. 2 illustrates the handle 18 and connected body portion 14 of the prior art device of Fig. 1.
Fig. 2A illustrates the prior art handle device 30 of Fig. 1.
Fig. 3 illustrates the exterior surface of the spindle-type surgical tool holder of the present invention.
Fig. 4 illustrates a cross-sectional view of figure 3 taken along the lines 4-4 and rotated 90 degrees.
Fig. 5 illustrates an exploded view of Fig. 3.
Fig. 6 illustrates Fig. 3 expanded for cleaning purposes.

### DETAILED DESCRIPTION OF THE INVENTION

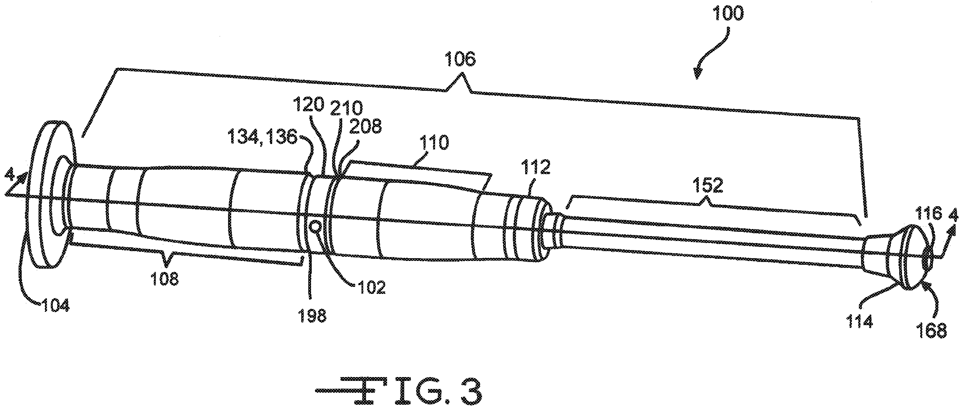

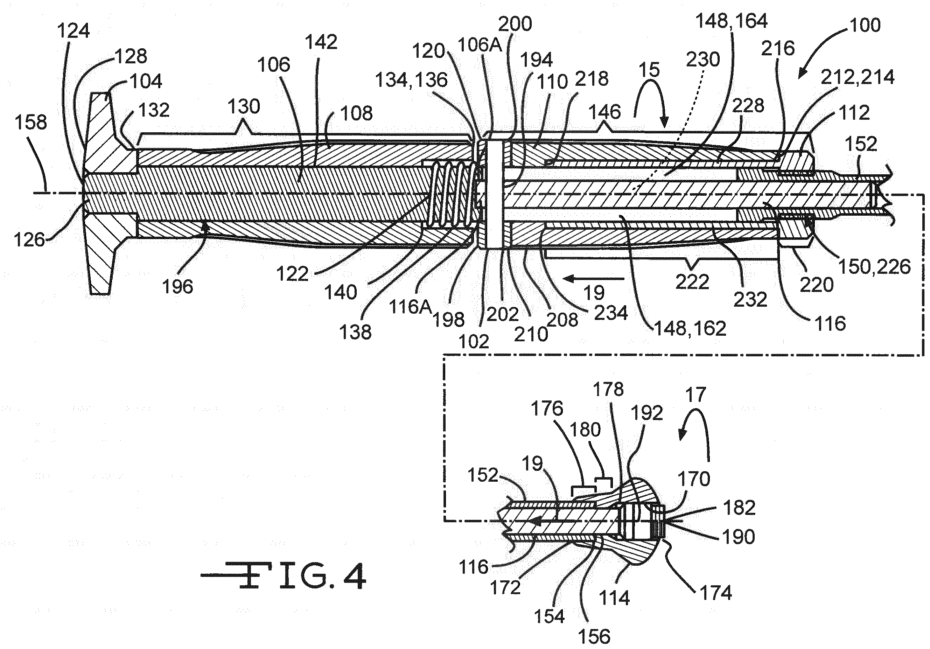

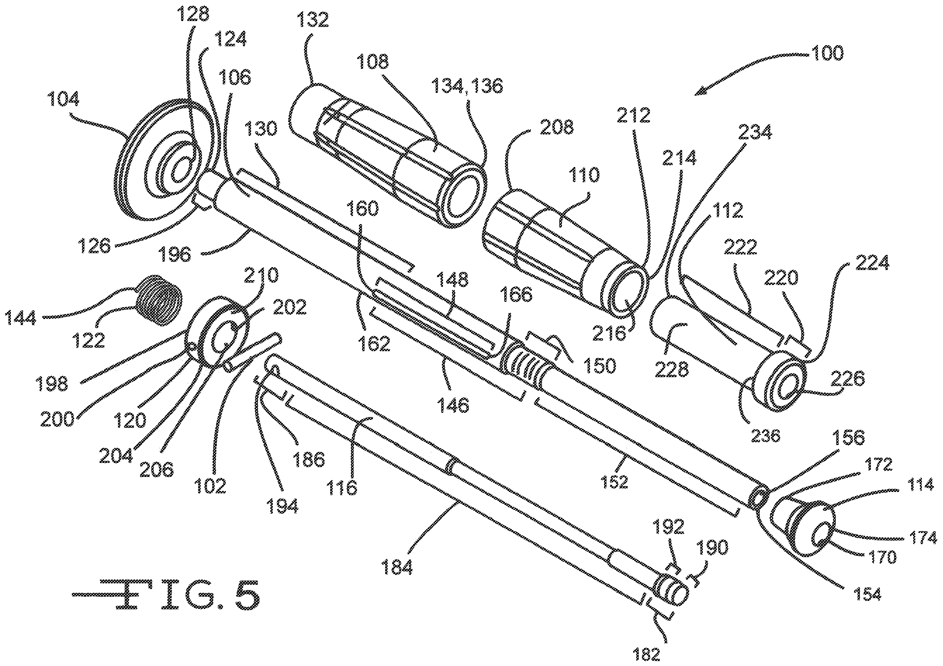

Figs. 3, 4 and 5 illustrate an improved spindle-type surgical tool holder 100 according to the present invention. Fig. 3 illustrates the exterior surface of the tool holder 100; Fig. 4 illustrates a cross-sectional view of Fig. 3 taken along the lines 4-4 and rotated 90 degrees so that shaft pin 102 is vertical instead of horizontal as in Fig. 3; and Fig. 5 illustrates an exploded view of Fig. 3. From that general understanding of FIGs. 3 to 5, we will discuss the spindle-type surgical tool holder 100.

The improved spindle-type surgical tool holder 100 has a strike plate 104, a body 106, a proximal fixed handle 108, a distal rotate handle 110, an insert handle with threads 112, an impactor head 114, a shaft 116, the shaft pin 102, a shaft ring 120, and a spring or any other conventional resilient member 122 (seen in Figs. 4 and 5).

The strike plate 104 connects to the proximal end 124 of the body 106. As illustrated, the strike plate 104 is press-fitted, welded, or by any other conventional means is securely attached to the body 106; or alternatively, it is an integral part of the body. In the embodiment illustrated in Figs. 4 and 5, the body 106 has a strike plate extension 126 at its proximal end 124 that press fits within the strike plate's female aperture 128. In any embodiment, the strike plate 104 is securely attached to the body 106 in such a way that the strike plate 104 is not easily removed, under normal operating conditions for the surgical tool holder.

The proximal fixed handle 108 extends from the strikeplate 104 and is positioned over a fixed handle area 130 of the body 106 (Figs. 4 to 5). The proximal fixed handle 108, as with the prior art tool holder 10, has a length that allows a surgeon to hold it, in one embodiment with one hand, and in an alternative embodiment with two hands. The fixed handle's 108 proximal end 132, in a preferred embodiment, contacts the strike plate 104. The fixed handle's 108 distal end 134 has a ring stopper distal end 136 (Figs. 3, 4 and 5), an internal spring cavity 138, a spring abutment 140 and an internal body cavity 142. The body cavity 142 extends from the spring abutment 140 to the proximal end 132 and has a diameter that allows it to be positioned over and preferably contact the fixed handle area 130 of the body 106. The spring cavity 138 is an opening adjacent to the body 106 that receives the body 106 and the spring 122 so the spring 122 can expand and contract depending on the force applied to it. The spring's tension force is sufficient to ensure that the surgical implant device 26 is securely attached, when desired, to the surgical tool holder 100. The spring abutment 140 is where the spring's proximal end 144 contacts the fixed handle 108.

As illustrated in Figs. 4 and 5, the body 106 has numerous sections. The first section is the strike plate extension 126 which has already been discussed. The second section is the fixed handle area 130 which has also been discussed. The third section is a rotating handle 110/ring 120 area 146 (Fig. 5). Within the rotating handle 110/ring 120 area 146, and in some embodiments an overlap in the fixed handle area 130, is a slot area 148 (Figs. 4 and 5). Also within the rotating handle/ring area 146 and at its distal end is a distal rotating thread area 150. A fourth section is an insertion area 152 that extends to the distal end 154 of the body 106.

Starting at the distal end 154, the body 106 has a cavity 156 of a diameter greater than the outer diameter of the shaft 116. That is so the shaft 116 can move in the cavity 156 along the body's and cavity's co-longitudinal axis 158 (Fig. 4). The cavity 156 extends proximally beyond the proximal end 160 (Fig. 5) of the slot area 148.

The slot area 148 contains a first axial opening 162 (Figs. 4 and 5) and, in most embodiments, a second axial opening 164 (Fig. 4) that is diametrically opposed to the first opening 162. The first and second axial openings 162, 164 receive the opposed ends of the shaft pin 102. Each axial opening 162, 164 has a predetermined length that allows the shaft pin 102, the shaft 116, and the shaft ring 120 to move a predetermined distance along the spindle's axis 158 to allow the surgical tool holder 100 to be easily cleaned, but securely attached to a surgical implant 26.

The distal rotating threaded area 150 is spaced a predetermined distance from the slot area's 148 distal end 166. That distance depends on the lengths of the distal rotate handle 110 and the insert handle with threads 112. The rotating thread area 150 has threads that mate with corresponding threads on the insert handle 112. The rotating thread area 150 has a length that allows the insert handle 112 to be rotated, through the distal rotate handle 110, to properly position the shaft ring 120, the shaft 116, and the surgical implant device 26 in order to decrease the chance of damaging or displacing the surgical implant 26 from the holder 100 during the surgical operation.

Extending from the rotating thread area 150 toward the body's distal end 154 is the insertion area 152. The insertion area 152 can be any desired length that assists and allows the surgeon to properly use the spindle-type surgical tool holder 100. Attached to the body's distal end 154 is the impactor head 114.

The impactor head 114 has a contacting surface 168 (Fig. 3), and a second cavity 170 that extends from the head's proximal end 172 to its distal end 174 along a second longitudinal axis, which is co-axial the body's longitudinal axis 158. The second cavity 170 has three sections as illustrated at Fig. 4. They are a body cavity 176, a tip cavity 178, and an interference fit cavity 180. The body cavity 176 has a first diameter that receives the body's distal end 154 and a length that impairs the impactor head 114 from disconnecting from the body's distal end 154.

The interference fit cavity 180 has a second diameter, smaller than the first body cavity diameter, which receives the shaft 116, but not the body's distal end 154. The shaft 116 is capable of movement along the interference fit cavity. The interference fit cavity 180 also creates an obstacle that inhibits the threaded distal end 182 of the shaft 116 from entering the body cavity 176. The tip cavity 178 has a third diameter, greater than the second diameter, and equal to, smaller than, or greater than the first diameter. The third diameter is sufficient to allow the shaft's 116 threaded distal end 182 to move therein and extend from the head's distal end 174.

The contacting surface 168 is at the head's distal end 174. The contacting surface 168 is designed to contact the interior surface of the surgical implant 26 (similar to how the prior art's implant support 34 operates). In that respect, the contacting surface 168 is designed to provide an additional stabilization surface to the surgical implant device 26 during the surgical procedure and to inhibit the implant 26 from disconnecting from the spindle-type surgical tool holder 100.

As shown in Fig. 5, the shaft 116 has three sections. They are the partially threaded distal end 182, an elongated intermediate section 184, and a proximal pin section 186. The threaded distal end 182 has at least a distal threaded area 190 having a first shaft diameter and an implant stopper area 192 having a second diameter, greater than or equal to the first shaft diameter. The distal threaded area 190 is designed to be threaded into the threaded aperture 28 of the surgical implant 26. Likewise, the implant stopper area 192 is designed to inhibit the surgical implant 26 from penetrating beyond the proximal end of the threaded area 190.

Extending in the proximal direction of the shaft 116 from the threaded distal end 182 is the elongated intermediate section 184. The elongated section can be a single diameter or multiple diameters (as illustrated in Figs. 4 and 5). In any event, the elongated section's diameter, which is smaller than that of the shaft cavity 156, enables movement of the elongated section 184 along the axis 158 of the cavity 156 and body 106.

Adjacent to the shaft's 116 proximal end is the pin section 186. The pin section 186 has a shaft aperture 194 that extends from one side to the opposing side of the shaft as illustrated in Figs. 4 and 5; but alternatively the aperture 194 could just be on the shaft's one side. While in the body's cavity, the shaft aperture 194 is aligned with the first and/or second axial openings 162 and 164 in the body 106.

The shaft aperture 194 has a diameter that receives and, possibly, secures the shaft pin 102 in place. The aperture and pin relationship can be a loose fit, a press-fit, a conventional rib and indent system, a conventional spring-loaded ball and indent system, or any functionally equivalent system that fixedly attaches the pin in the aperture or securely attaches, and also allows the release of, the pin in the aperture, or alternatively allows the pin 102 to move freely within the aperture 194. The aperture and pin relationship depends on how the pin 102 is used in association with other components of the present invention. For example, if there is no shaft ring 120, the pin and aperture have to be permanently attached or securely attached. Likewise, if there is a shaft ring 120, then the pin and aperture can be any of the three possible attachment embodiments especially if the shaft ring and the pin are securely attached to each other.

Prior to inserting the pin 102 in the aperture 194 and through the shaft's first and second axial openings 162 and 164, the shaft ring 120 is properly positioned on the shaft's exterior surface 196. As previously stated, the spring 122 is positioned in the fixed handle's 108 spring cavity 138 with its proximal end 144 contacting the spring abutment 140. Likewise, the proximal end 198 of the shaft ring 120, preferably contacts the fixed handle's ring stopper distal end 136. In addition, the shaft ring 120 has one or, preferably, two pin apertures 200, 202 that are diametrically opposed to each other. Each pin aperture extends from the shaft ring's outer surface 204 to its respective inner surface 206 and has a diameter that receives and secures the pin 102 in place. The pin apertures 200, 202 and shaft pin 102 relationship can be a press-fit, a rib and indent system, a spring-loaded ball and indent system, or any functionally equivalent system that either permanently attaches the shaft pin in the apertures 200, 202 or securely attaches, and also allows the release of, the pin 102 in the apertures. As expected, the pin apertures 200, 202, the shaft aperture 194 and the shaft's 116 first and second axial openings 162 and 164 must be aligned to receive the shaft pin 102.

Prior to or after the shaft pin 102 is properly positioned and secured in the apertures 200, 202, the shaft aperture 194 and the first and second axial shaft openings 162 and 164, and the rotate handle 110 are positioned over a portion of the exterior surface of the insert handle with threads 112. This assembly is then positioned over the rotating handle 110/ring 120 area's 146 exterior surface 196. The distal rotating handle 110 has a proximal end 208 that contacts the distal end 210 of the shaft ring 120, and a distal end 212. The distal end 212 has a threaded insert handle stopper 214, an insert handle cavity 216 (Fig. 4 and 5) that extends to the proximal end 208, and an insert handle cavity stopper 218 (Fig. 4).

The insert handle 112 has a distal threaded area 220 and a proximal rotating area 222. The threaded area 220 has an outer diameter 224 (Fig. 5) and an inner threaded diameter 226 (Figs. 4 and 5). The inner threaded diameter 226 has threads that threadingly engage with the rotating thread area 150. The proximal rotating area 222 has an outer diameter 228, preferably smaller than the threaded area's outer diameter 224, and an inner diameter 230. In order for the rotating area 222 to freely rotate, the inner diameter 230 is greater than the outer diameter 232 of the rotating handle area 110 of the body. The insert handle cavity 216 receives the rotating area 222 of the insert handle 112 so that its proximal end 234 contacts the insert handle cavity stopper 218; and the threaded area's 220 proximal end 236 contacts the threaded insert handle stopper 214.

In use, when a surgical implant device 26 is threaded onto the shaft's threaded area 190, the surgeon merely rotates the assembly comprising the distal rotate handle 110 and the insert handle with threads 112 in one direction, normally clockwise (see arrow 15 in Fig. 4). This movement causes the distal threaded area 190 of the shaft 116 to rotate in a second, opposite direction, normally counterclockwise (see arrow 17), to act as a lock-nut for the surgical implant 26. This movement simultaneously pulls the shaft 116 (see arrow 19 in Fig. 4) toward the spindle-type surgical tool holder 100 proximal end 124. The pulling motion ensures the surgical implant device is positioned against the impactor head's contacting surface 168.

Then, in order to position an implant 26 in an acetabulum, an axial force is imparted to the strike plate 104 connected to the body 106. The body 106 housed inside the proximal fixed handle 108 is, in turn, rigidly connected to the shaft 116 having its proximal end 116A seated in a distal recess 106A of the body 106. The opposite, distal end of the shaft is threaded into the implant 26. That way, the entire assembly is static or rigid with the implant attached held firmly against impactor head 114. This provides a good tactile feel to the surgeon as the force imparted to the strike plate 104 translates through the tool holder 100 to effect implantation of the cup 26.

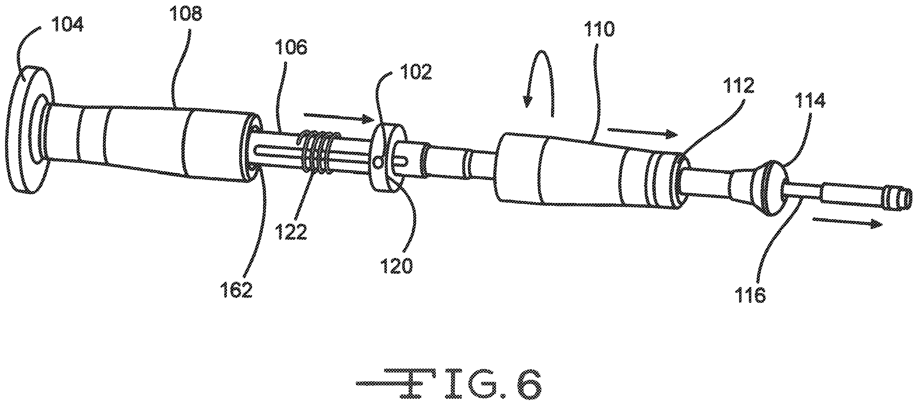

As illustrated at Fig. 6, cleaning of the claimed spindle-type surgical tool holder 100 is also relatively easy. First, the user disengages the inner threaded diameter 226 from the rotating thread area 150. Second, the user pulls all the moving components toward the holder's distal end, to expose them for easy cleaning.

Although several embodiments of the invention have been described in detail for purposes of illustration, various modifications of each may be made without departing from the scope of the invention. For example, there can be just one opening or a plurality of openings for the slot area. The shaft pin may only extend from one opening or two openings. In addition, there may be numerous shaft pins extending from one opening or two openings in the slot area. The resilient member can be a spring of any conventional resilient object made of metal, plastic, or a gelatinous elastomeric material made from an A-B-A triblock copolymer. Examples of A-B-A triblock copolymers are SEP, SEB, SEBS, SEEPS ("S" is styrene monomer(s), "E" is ethylene monomer(s), "B" is butylene monomer(s), and "P" is propylene monomer(s)) and variations thereof made by the Kuraray Corporation. The directions of clockwise and counterclockwise can be transposed depending on the thread types. Depending on the shape of the shaft pin, the shaft ring is optional. Accordingly, the invention is not limited, except by the appended claims.

## Claims

1. A surgical tool handling device comprising:
a body (106) comprising (a) a longitudinal axis (158), (b) a cavity (156) that extends from the body's distal end (154) toward the body's proximal end (124) and has a first diameter that allows a shaft's (116) elongated section (184) and proximal end (186) to move along the body's longitudinal axis (158), (c) a first axial opening (162) positioned on the body's exterior surface that (i) extends a predetermined length along the body's longitudinal axis (158) so it has an axial opening's proximal end (160) and an axial opening's distal end (166), (ii) extends from the body's exterior surface to the cavity (156), and (iii) has a width that allows a shaft pin (102) to move along the body's longitudinal axis (158), and (d) a rotating thread area (150) positioned on the body's exterior surface between the distal end (154) and the axial opening's distal end (166);
an impactor head (114) having a second longitudinal axis, positioned at the body's distal end (154), having a second cavity (170) extending from the impactor head's distal end (174) to the impactor head's proximal end (172) along the second longitudinal axis to allow the shaft's elongated section (184) and distal end (182) to have movement along the second longitudinal axis, and having a medical instrument stabilizing exterior surface (168) positioned near the distal end's second cavity (170) ;
a shaft (116) having a first shaft aperture (194) near or toward the shaft's proximal end (186), a threaded distal end (182) positioned within, at or extending from the impactor head's distal end (174), and an elongated section (184) positioned between the first shaft aperture (194) and the threaded distal end (182);
a shaft pin having a distal end which protrudes from the first axial opening (162) while a section of the shaft pin (102) positioned toward the shaft pin's proximal end in relation to the distal end is securely positioned in the shaft aperture (194);
a rotating, adjusting system (146) positioned on the body's exterior surface, wherein the rotating, adjusting system's distal section has an interior surface having a second threaded area (220), and the second threaded area (220) engages with the body's rotating thread area (150);
a spring (122) received in a spring cavity (138);
a strikeplate (104) positioned at the body's proximal end (124); and
a fixed handle (108) positioned between the strikeplate (104) and the shaft pin (102), wherein the fixed handle (108) comprises the spring cavity (138) which is an opening adjacent to the body (106) that receives the body (106) and the spring (122) so the spring (122) can expand and contract depending on the force applied to it;
wherein the body's rotating thread area (150) and the second threaded area (220) in relation to the shaft's threaded distal end (182) are independent from each other;
wherein a medical instrument can be threadingly interconnected to the threaded distal end (182) when a portion of the threaded distal end (182) extends beyond the impactor head's distal end (174); and
wherein when the rotating, adjusting system's threaded area (220) engages the body's rotating thread area (150) and the rotating, adjusting system (146) is rotated in a first direction, the shaft (116) is pulled toward the body's proximal end (124).

2. The surgical tool handling device of claim 1 wherein the medical instrument stabilizing exterior surface (168) is positioned around the distal end's second cavity (170).

3. The surgical tool handling device of claim 1 or claim 2 wherein the shaft pin's distal end interconnects to a shaft ring (120).

4. The surgical tool handling device of any of claims 1 to 3 wherein the body (106) further comprises a second axial opening (164) positioned on the body's exterior surface.

5. The surgical tool handling device of claim 4 wherein the second axial opening (164) is diametrically opposed to the first axial opening (162).

6. The surgical tool handling device of claim 4 or claim 5 wherein the shaft pin's proximal end protrudes from the second axial opening (164).

7. The surgical tool handling device of any of claims 1 to 6 wherein the shaft's threaded distal end (182) has a diameter greater than the shaft's elongated section (184), and the impactor head's second cavity (170) has an impedance barrier (180) that inhibits the shaft's threaded distal end (182) from entering the body's cavity (156).

8. A kit comprising:
a medical instrument having a threaded aperture; and
a surgical tool handling device as defined in any of claims 1 to 7.

## Patentansprüche

1. Chirurgische Werkzeughandhabungsvorrichtung, die Folgendes umfasst:
ein Gehäuse (106) mit: (a) einer Längsachse (158), (b) einem Hohlraum (156), der vom distalen Ende (154) des Gehäuses in Richtung proximales Ende (124) des Gehäuses verläuft und einen ersten Durchmesser hat, der es zulässt, dass sich ein länglicher Abschnitt (184) eines Schafts (116) und ein proximales Ende (186) entlang der Längsachse (158) des Gehäuses bewegen, (c) einer ersten axialen Öffnung (162), die an der Außenfläche des Gehäuses positioniert ist, die (i) über eine vorbestimmte Länge entlang der Längsachse (158) des Gehäuses verläuft, so dass sie ein proximales Ende (160) einer axialen Öffnung und ein distales Ende (166) einer axialen Öffnung hat, (ii) von der Außenfläche des Gehäuses zum Hohlraum (156) verläuft und (iii) eine Breite hat, die es zulässt, dass sich ein Schaftbolzen (102) entlang der Längsachse (158) des Gehäuses bewegt, und (d) einem drehbaren Gewindebereich (150), der an der Außenfläche des Gehäuses zwischen dem distalen Ende (154) und dem distalen Ende (166) der axialen Öffnung positioniert ist;
einen Schlagkopf (114) mit einer zweiten Längsachse, positioniert am distalen Ende (154) des Gehäuses, mit einem zweiten Hohlraum (170), der vom distalen Ende (174) des Schlagkopfs zum proximalen Ende (172) des Schlagkopfs entlang der zweiten Längsachse verläuft, um es zuzulassen, dass der längliche Abschnitt (184) des Schafts und das distale Ende (182) Bewegung entlang der zweiten Längsachse haben, und mit einer das medizinische Instrument stabilisierenden Außenfläche (168), die nahe dem zweiten Hohlraum (170) des distalen Endes positioniert ist;
einen Schaft (116) mit einer ersten Schaftöffnung (194) nahe oder in Richtung des proximalen Endes (186) des Schafts, ein distales Gewindeende (182), das in, an oder ausgehend von dem distalen Ende (174) des Schlagkopfs positioniert ist, und einen länglichen Abschnitt (184), der zwischen der ersten Schaftöffnung (194) und dem distalen Gewindeende (182) positioniert ist;
einen Schaftbolzen mit einem distalen Ende, das von der ersten axialen Öffnung (162) vorsteht, während ein Abschnitt des Schaftbolzens (102), positioniert in Richtung proximales Ende des Schaftbolzens, in Bezug auf das distale Ende sicher in der Schaftöffnung (194) positioniert ist;
ein drehbares Justiersystem (146), positioniert an der Außenfläche des Gehäuses, wobei der drehbare distale Abschnitt des Justiersystems eine Innenfläche mit einem zweiten Gewindebereich (220) hat und der zweite Gewindebereich (220) in den drehbaren Gewindebereich (150) des Gehäuses eingreift;
eine in einem Federhohlraum (138) aufgenommene Feder (122) ;
eine Anschlagplatte (104), die am proximalen Ende (124) des Gehäuses positioniert ist; und
einen festen Griff (108), der zwischen der Anschlagplatte (104) und dem Schaftbolzen (102) positioniert ist, wobei der feste Griff (108) den Federhohlraum (138) umfasst, der eine Öffnung neben dem Gehäuse (106) ist, die das Gehäuse (106) und die Feder (122) aufnimmt, so dass sich die Feder (122) je nach der darauf aufgebrachten Kraft ausdehnt und zusammenzieht;
wobei der drehbare Gewindebereich (150) des Gehäuses und der zweite Gewindebereich (220) in Bezug auf das distale Gewindeende (182) des Schafts unabhängig voneinander sind;
wobei ein medizinisches Instrument mit dem distalen Gewindeende (182) zusammengeschraubt werden kann, wenn ein Teil des distalen Gewindeendes (182) über das distale Ende (174) des Schlagkopfs hinaus verläuft; und
wobei der Schaft (116), wenn der drehbare Gewindebereich (220) des Justiersystems in den drehbaren Gewindebereich (150) des Gehäuses eingreift und das drehbare Justiersystem (146) in einer ersten Richtung gedreht wird, in Richtung proximales Ende (124) des Gehäuses gezogen wird.

2. Chirurgische Werkzeughandhabungsvorrichtung nach Anspruch 1, wobei die das medizinische Instrument stabilisierende Außenfläche (168) um den zweiten Hohlraum (170) des distalen Endes herum positioniert ist.

3. Chirurgische Werkzeughandhabungsvorrichtung nach Anspruch 1 oder Anspruch 2, wobei das distale Ende des Schaftbolzens mit einem Schaftring (120) verbunden ist.

4. Chirurgische Werkzeughandhabungsvorrichtung nach einem der Ansprüche 1 bis 3, wobei das Gehäuse (106) ferner eine zweite axiale Öffnung (164) aufweist, die um die Außenfläche des Gehäuses herum positioniert ist.

5. Chirurgische Werkzeughandhabungsvorrichtung nach Anspruch 4, wobei die zweite axiale Öffnung (164) der ersten axialen Öffnung (162) diametral gegenüberliegt.

6. Chirurgische Werkzeughandhabungsvorrichtung nach Anspruch 4 oder Anspruch 5, wobei das proximale Ende des Schaftbolzens von der zweiten axialen Öffnung (164) vorsteht.

7. Chirurgische Werkzeughandhabungsvorrichtung nach einem der Ansprüche 1 bis 6, wobei das distale Gewindeende (182) des Schafts einen Durchmesser hat, der größer ist als der längliche Abschnitt (184) des Schafts, und der zweite Hohlraum (170) des Schlagkopfs eine Impedanzbarriere (180) hat, die verhindert, dass das distale Gewindeende (182) des Schafts in den Hohlraum (156) des Körpers eintritt.

8. Kit, der Folgendes umfasst:
ein medizinisches Instrument mit einer Gewindeöffnung; und
eine chirurgische Werkzeughandhabungsvorrichtung nach einem der Ansprüche 1 bis 7.

## Revendications

1. Dispositif de manipulation d'outil chirurgical comprenant :
un corps (106) comprenant (a) un axe longitudinal (158), (b) une cavité (156) qui s'étend depuis l'extrémité distale du corps (154) vers l'extrémité proximale du corps (124) et a un premier diamètre qui permet à une section allongée (184) et à une extrémité proximale(186) d'un arbre (116) de se déplacer le long de l'axe longitudinal du corps (158), (c) une première ouverture axiale (162) positionnée sur la surface extérieure du corps qui (i) s'étend sur une longueur prédéterminée le long de l'axe longitudinal du corps (158) de sorte qu'elle a une extrémité proximale de l'ouverture axiale (160) et une extrémité distale de l'ouverture axiale (166), (ii) s'étend depuis la surface extérieure du corps vers la cavité (156), et (iii) a une largeur qui permet à une tige d'arbre (102) de se déplacer le long de l'axe longitudinal du corps (158), et (d) une zone de filetage rotative (150) positionnée sur la surface extérieure du corps entre l'extrémité distale (154) et l'extrémité distale de l'ouverture axiale (166) ;
une tête d'impacteur (114) ayant un deuxième axe longitudinal, positionnée au niveau de l'extrémité distale du corps (154), ayant une deuxième cavité (170) s'étendant depuis l'extrémité distale de la tête d'impacteur (174) vers l'extrémité proximale de la tête d'impacteur (172) le long du deuxième axe longitudinal pour permettre à la section allongée (184) et à l'extrémité distale (182) de l'arbre d'effectuer un mouvement le long du deuxième axe longitudinal, et ayant une surface extérieure stabilisant un instrument médical (168) positionnée près de la deuxième cavité de l'extrémité distale (170) ;
un arbre (116) ayant un premier orifice d'arbre (194) près de ou vers l'extrémité proximale de l'arbre (186), une extrémité distale filetée (182) positionnée au sein de, au niveau de ou s'étendant depuis l'extrémité distale de la tête d'impacteur (174), et une section allongée (184) positionnée entre le premier orifice d'arbre (194) et l'extrémité distale filetée (182) ;
une tige d'arbre ayant une extrémité distale qui fait saillie depuis la première ouverture axiale (162) alors qu'une section de la tige d'arbre (102) positionnée vers l'extrémité proximale de la tige d'arbre en relation avec l'extrémité distale est positionnée solidement dans l'orifice d'arbre (194) ;
un système d'ajustement, rotatif, (146) positionné sur la surface extérieure du corps, dans lequel la section distal du système d'ajustement, rotatif, a une surface intérieure ayant une deuxième zone filetée (220), et la deuxième zone filetée (220) entre en prise avec la zone de filetage rotative du corps (150) ;
un ressort (122) reçu dans une cavité de ressort (138) ;
une gâche (104) positionnée au niveau de l'extrémité proximale du corps (124) ; et
une poignée fixe (108) positionnée entre la gâche (104) et la tige d'arbre (102), dans lequel la poignée fixe (108) comprend la cavité de ressort (138) qui est une ouverture adjacente au corps (106) qui reçoit le corps (106) et le ressort (122) de sorte que le ressort (122) peut se déployer et se contracter en fonction de la force appliquée sur celui-ci ;
dans lequel la zone de filetage rotative du corps (150) et la deuxième zone filetée (220) en relation avec l'extrémité distale filetée de l'arbre (182) sont indépendantes l'une de l'autre ;
dans lequel un instrument médical peut être interconnecté par filetage avec l'extrémité distale filetée (182) quand une partie de l'extrémité distale filetée (182) s'étend au-delà de l'extrémité distale de la tête d'impacteur (174) ; et
dans lequel quand la zone filetée du système d'ajustement, rotatif (220) entre en prise avec la zone de filetage rotative du corps (150) et le système d'ajustement, rotatif (146) est amené à tourner dans une première direction, l'arbre (116) est tiré vers l'extrémité proximale du corps (124).

2. Dispositif de manipulation d'outil chirurgical selon la revendication 1 dans lequel la surface extérieure stabilisant un instrument médical (168) est positionnée autour de la deuxième cavité de l'extrémité distale (170).

3. Dispositif de manipulation d'outil chirurgical selon la revendication 1 ou la revendication 2 dans lequel l'extrémité distale de la tige d'arbre interconnecte avec une bague d'arbre (120).

4. Dispositif de manipulation d'outil chirurgical selon l'une quelconque des revendications 1 à 3 dans lequel le corps (106) comprend en outre une deuxième ouverture axiale (164) positionnée sur la surface extérieure du corps.

5. Dispositif de manipulation d'outil chirurgical selon la revendication 4 dans lequel la deuxième ouverture axiale (164) est diamétralement opposée à la première ouverture axiale (162).

6. Dispositif de manipulation d'outil chirurgical selon la revendication 4 ou la revendication 5 dans lequel l'extrémité proximale de la tige d'arbre fait saillie depuis la deuxième ouverture axiale (164).

7. Dispositif de manipulation d'outil chirurgical selon l'une quelconque des revendications 1 à 6 dans lequel l'extrémité distale filetée de l'arbre (182) a un diamètre supérieur à la section allongée de l'arbre (184), et la deuxième cavité de la tête d'impacteur (170) a une barrière d'impédance (180) qui empêche l'extrémité distale filetée de l'arbre (182) de pénétrer dans la cavité du corps (156).

8. Trousse comprenant :
un instrument médical ayant une ouverture filetée ; et
un dispositif de manipulation d'outil chirurgical tel que défini dans l'une quelconque des revendications 1 à 7.
